# EUROPEAN PATENT SPECIFICATION

(11) **EP 2 486 806 B1**
(45) Date of publication and mention of the grant of the patent: **28.11.2018**
(21) Application number: 11006390.6
(22) Date of filing: 03.08.2011
(51) Int. Cl.: A23L 2/60, A61K 8/33, A23L 33/10, A23L 27/10, A23L 27/30

(54) **Stevia composition**
Stevia-Zusammensetzung
Composition de Stevia

(30) Priority: 10.02.2011 US 441443 P; 11.05.2011 WO PCT/US2011/036063
(43) Date of publication of application: 15.08.2012
(73) Proprietor: Purecircle USA, Chicago, IL 60606 (US)
(72) Inventor: Markosyan, Avetik A., Yerevan 0014 (AM)
(74) Representative: Vossius & Partner Patentanwälte Rechtsanwälte mbB

(56) References cited:
- WO-A1-2012/082677
- WO-A2-2012/125991
- US-A1- 2007 116 836
- US-A1- 2010 112 154
- US-A1- 2010 316 782

## Description

### BACKGROUND

### Field Of The Invention

The invention relates to a process for producing a rebaudioside B composition from the extract of the *Stevia rebaudiana* Bertoni plant, as characterized by the appended claims.

### Description Of The Related Art

In this specification, a number of documents including patent applications and manufacturer's manuals is cited.

Nowadays sugar alternatives are receiving increasing attention due to awareness of many diseases in conjunction with consumption of high-sugar foods and beverages. However many artificial sweeteners such as dulcin, sodium cyclamate and saccharin were banned or restricted in some countries due to concerns on their safety. Therefore non-caloric sweeteners of natural origin are becoming increasingly popular. The sweet herb *Stevia rebaudiana* Bertoni, produces a number of diterpene glycosides which feature high intensity sweetness and sensory properties superior to those of many other high potency sweeteners.

The above-mentioned sweet glycosides, have a common aglycon, steviol, and differ by the number and type of carbohydrate residues at the C13 and C19 positions. The leaves of Stevia are able to accumulate up to 10-20% (on dry weight basis) steviol glycosides. The major glycosides found in Stevia leaves are rebaudioside A (2-10%), stevioside (2-10%), and rebaudioside C (1-2%). Other glycosides such as rebaudioside *B*, *D, E,* and *F*, steviolbioside and rubusoside are found at much lower levels (approx. 0-0.2%).

Two major glycosides - stevioside and rebaudioside *A* (reb *A*), were extensively studied and characterized in terms of their suitability as commercial high intensity sweeteners. Stability studies in carbonated beverages confirmed their heat and pH stability (Chang S.S., Cook, J.M. (1983) Stability studies of stevioside and rebaudioside A in carbonated beverages. J. Agric. Food Chem. 31: 409-412.)

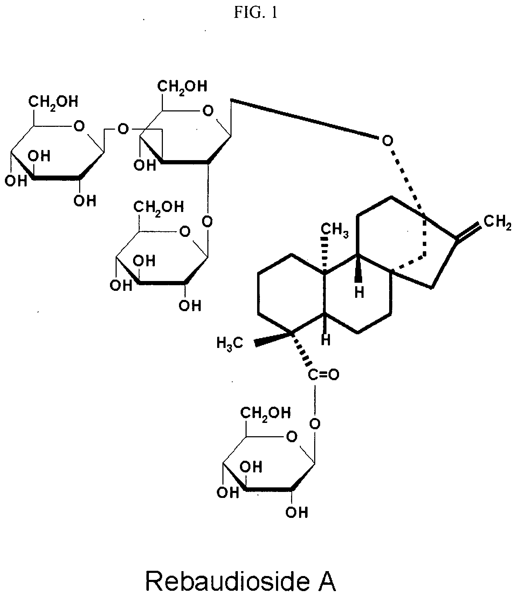

Steviol glycosides differ from each other not only by molecular structure, but also by their taste properties. Usually stevioside is found to be 110-270 times sweeter than sucrose, rebaudioside *A* between 150 and 320 times, and rebaudioside *C* between 40-60 times sweeter than sucrose. Dulcoside *A* is 30 times sweeter than sucrose. Rebaudioside *A* has the least astringent, the least bitter, and the least persistent aftertaste thus possessing the most favorable sensory attributes in major steviol glycosides (Tanaka O. (1987) Improvement of taste of natural sweetners. Pure Appl. Chem. 69:675-683; Phillips K.C. (1989) Stevia: steps in developing a new sweetener. In: Grenby T.H. ed. Developments in sweeteners, vol. 3. Elsevier Applied Science, London. 1-43.) The chemical structure of rebaudioside *A* is shown in Fig. 1.

Methods for the extraction and purification of sweet glycosides from the *Stevia rebaudiana* plant using water or organic solvents are described in, for example, U.S. Patent Numbers 4,361,697; 4,082,858; 4,892,938; 5,972,120; 5,962,678; 7,838,044 and 7,862,845.

However, even in a highly purified state, steviol glycosides still possess undesirable taste attributes such as bitterness, sweet aftertaste, licorice flavor, etc. One of the main obstacles for the successful commercialization of stevia sweeteners are these undesirable taste attributes. It was shown that these flavor notes become more prominent as the concentration of steviol glycosides increases (Prakash I., DuBois G.E., Clos J.F., Wilkens K.L., Fosdick L.E. (2008) Development of rebiana, a natural, non-caloric sweetener. Food Chem. Toxicol., 46, S75-S82.).

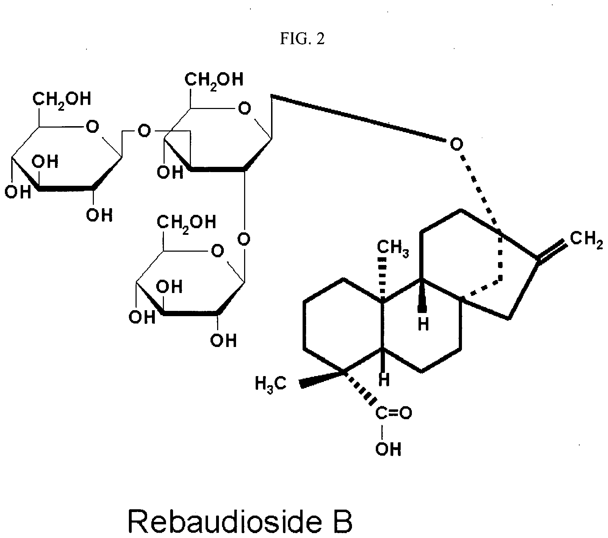

Rebaudioside *B* (CAS No: 58543-17-2), or reb *B,* also known as stevioside *A₄* (Kennelly E.J. (2002) Constituents of Stevia rebaudiana In Stevia: The genus Stevia, Kinghorn A.D. (Ed), Taylor & Francis, London, p.71), is one of the sweet glycosides found in *Stevia rebaudiana.* Sensory evaluations show that reb *B* was approximately 300-350 times sweeter than sucrose, while for reb *A* this value was approximately 350-450 (Crammer, B. and Ikan, R. (1986) Sweet glycosides from the Stevia plant. Chemistry in Britain 22, 915-916, and 918). The chemical structure of rebaudioside *B* is shown in Fig.2.

It was believed that reb *B* forms from partial hydrolysis of rebaudioside *A* during the extraction process (Kobayashi, M., Horikawa, S., Degrandi, I.H., Ueno, J. and Mitsuhashi, H. (1977) Dulcosides A and B, new diterpene glycosides from Stevia rebaudiana. Phytochemistry 16, 1405-1408). However further research shows that reb *B* occurs naturally in the leaves of *Stevia rebaudiana* and currently it is one of nine steviol glycosides recognized by FAO/JECFA (United Nations' Food and Agriculture Organization/Joint Expert Committee on Food Additives) in calculating total steviol glycosides' content in commercial steviol glycosides preparations (FAO JECFA (2010) Steviol Glycosides, Compendium of Food Additive Specifications, FAO JECFA Monographs 10, 17-21).

Only a few methods are described in literature for preparing reb B.

Kohda et al., (1976) prepared reb *B* by hydrolysis of reb *A* with hesperidinase. Reb *B* was also prepared by alkaline saponification of reb *A.* The said saponification was conducted in 10% KOH-EtOH. The solution was acidified with AcOH, and extracted with n-BuOH. The BuOH-layer was washed with water and concentrated at low temperature *in vacuo.* The residue was crystallized from MeOH to give reb *B.* (Kohda, H., Kasai, R., Yamasaki, K., Murakami, K. and Tanaka, O. (1976) New sweet diterpene glucosides from Stevia rebaudiana. Phytochemistry 15, 981-983). The described processes might be suitable for laboratory scale preparation of reb *B*, but are hardly suitable for any large scale or commercial reb *B* preparation.

Ahmed et al., used mild alkaline hydrolysis of reb *A* to prepare reb *B* According to described procedure reb *A* was hydrolyzed to reb *B,* by refluxing with 10% aqueous KOH at 100°C for 1hr. After neutralization with glacial acetic acid, the precipitated substance was recrystallized twice from methanol (Ahmed M.S., Dobberstein R.H., and Farnsworth N.R. (1980) Stevia rebaudiana: I. Use of p-bromophenacyl bromide to enhance ultraviolet detection of water-soluble organic acids (steviolbioside and rebaudioside B) in high-performance liquid chromatographic analysis, J. Chromatogr., 192, 387-393). The use of methanol as recrystallization media will require its subsequent removal from the product. It is noted that handling of toxic substances such as methanol requires specialized manufacturing installations and, when applied in food processing, sophisticated food safety measures.

US 2007/0116836 describes functional sweetener compositions for the treatment and/or prevention of osteoporosis. US 2010/0316782 describes compositions that include rebaudioside D, processes to produce rebaudioside D, and formulations that provide ratios of Rebaudioside A to rebaudioside D to decrease the aftertaste of Rebaudioside A. US 2010/0112154 describes highly purified stevioside and Rebaudioside A prepared from sweet glycoside extracts obtained from *Stevia rebaudiana* Bertoni leaves.

It is also noted that no significant work has been conducted to determine the potential of reb *B* as a sweetener or food ingredient. Moreover reb *B* is often viewed as process artifact and unnecessary impurity in commercial steviol glycosides preparations. No significant evaluation of reb *B* influence on overall taste profile of steviol glycosides preparations has been conducted.

On the other hand, the water solubility of reb *B* is reported to be about 0.1% (Kinghorn A.D. (2002) Constituents of Stevia rebaudiana In Stevia: The genus Stevia, Kinghorn A.D. (Ed), Taylor & Francis, London, p.8). In many food processes where highly concentrated ingredients are used, a highly soluble form of reb *B* will be necessary.

Considering the facts mentioned above, it is necessary to evaluate reb *B* as a sweetener and food ingredient and develop a simple and efficient process for food grade reb *B* preparations suitable for food applications.

Within the present description we will show that, when applied in specific manner, reb *B* may impact the taste profile and offer significant advantages for stevia sweeteners' use in various applications.

### SUMMARY

The present invention is aimed to overcome the disadvantages of existing Stevia sweeteners. The invention describes a process for producing a rebaudioside B composition from the extract of the *Stevia rebaudiana* Bertoni plant, as characterized by the appended claims.

Also described herein is an ingredient comprising steviol glycosides of *Stevia rebaudiana* Bertoni plant. The steviol glycodsides are selected from the group consisting of stevioside, rebaudioside *A,* rebaudioside *B,* rebaudioside *C,* rebaudioside *D,* rebaudioside *E,* rebaudioside *F,* dulcoside *A,* steviolbioside, rubusoside, as well as other steviol glycosides found in *Stevia rebaudiana* Bertoni plant and mixtures thereof.

Also described herein is a process for producing an ingredient containing rebaudioside *B,* and stevioside, rebaudioside *A,* rebaudioside *C,* rebaudioside *D,* rebaudioside *E,* rebaudioside *F,* dulcoside *A,* steviolbioside, rubusoside, as well as other steviol glycosides found in *Stevia rebaudiana* Bertoni plant and mixtures thereof.

In the invention, rebaudioside *A* commercialized by PureCircle Sdn. Bhd. (Malaysia), containing, rebaudioside *A* (about 95-100%), stevioside (about 0-1%), rebaudioside *C* (about 0-1%), rebaudioside *F* (about 0-1%), rebaudioside *B* (about 0.1-0.8%), rebaudioside *D* (about 0-1%), and other glycosides amounting to total steviol glycosides' content of at least 95%, was used as a starting material. Alternatively stevia extracts with different ratio of steviol glycosides may be used as starting materials.

The starting material was subjected to partial conversion into reb B under elevated pH conditions in aqueous media free of any co-solvents, including toxic alkanols. The obtained glycoside mixtures were used "as-is" as well as reb *B* was recovered and used as a pure ingredient.

The low solubility reb *B* and mixtures thereof were subjected to additional thermal treatment to increase solubility.

The obtained products were applied in various foods and beverages as sweeteners, sweetener enhancers and flavor modifiers, including soft drinks, ice cream, cookies, bread, fruit juices, milk products, baked goods and confectionary products.

### BRIEF DESCRIPTION OF THE DRAWINGS

The accompanying drawings are included to provide a further understanding of the invention. The drawings illustrate embodiments of the invention and together with the description serve to explain the principles of the embodiments of the invention.
FIG. 1 shows the chemical structure of rebaudioside *A.*
FIG. 2 shows the chemical structure of rebaudioside *B.*

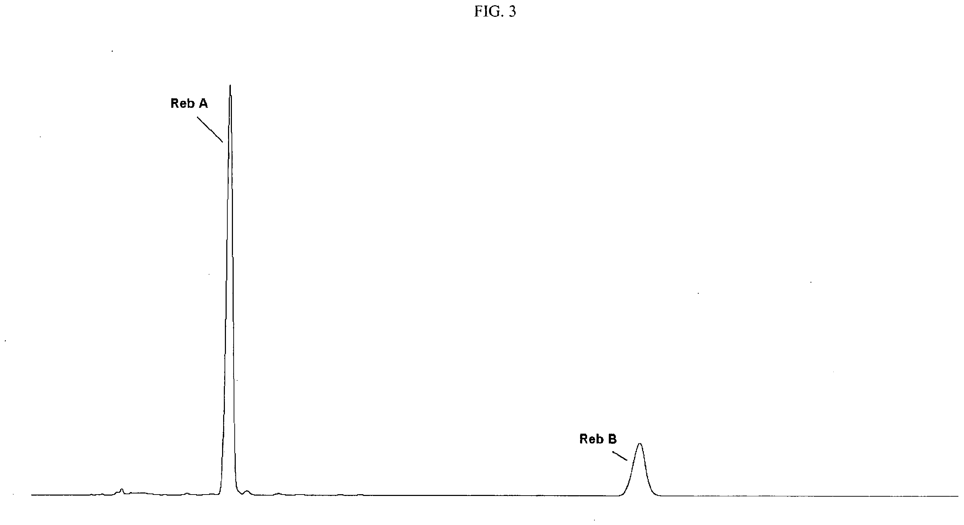

FIG. 3 shows an HPLC chromatogram of a stevia composition comprising rebaudioside *A* and rebaudioside *B.*

### DETAILED DESCRIPTION OF THE INVENTION

Advantages of the present invention will become more apparent from the detailed description given hereinafter.

Rebaudioside A commercialized by PureCircle Sdn. Bhd. (Malaysia), containing, rebaudioside A (about 95-100%), stevioside (about 0-1%), rebaudioside C (about 0-1%), rebaudioside *D* (about 0-1%), rebaudioside *F* (about 0-1%), rebaudioside *B* (about 0.1-0.8%) and other glycosides amounting to total steviol glycosides' content of at least about 95%, was used as a starting material. Alternatively stevia extracts with different ratio of steviol glycosides may be used as starting materials.

It will be appreciated by the skilled person that the term "about", as used herein, encompasses the explicitly recited values as well as small deviations therefrom. In other words, a content of "about 95%" does not have to be exactly the recited amount of 95% but may differ by several percent, thus including for example 92%, 93%, 94%, 96%, 97 % or 98%. The skilled person is aware that such values are relative values that do not require a complete accuracy as long as the values approximately corresponds to the recited values. Accordingly, a deviation from the recited value of for example 15%, more preferably of 10%, and most preferably of 5% is encompassed by the term "about".

The HPLC analysis of the raw materials and products was performed on an Agilent Technologies 1200 Series (USA) liquid chromatograph, equipped with Phenomenex Prodigy ODS3, 5 µm (4.6X250mm) column at 40°C. The mobile phase was 32:68 mixture of acetonitrile and 10 mmol/L sodium phosphate buffer (about pH 2.6) at 1 mL/min. A diode array detector set at 210 nm was used as the detector.

In the invention, reb *A* was dispersed in aqueous alkaline solution. The concentration of reb *A* is about 0-50% (w/v) preferably about 10-25%. The skilled person will appreciate that these ranges include all the values falling within the explicitly recited ranges, such as e.g. 5-40%, 12.5-20% or 15-17.5%. The preferred alkaline agents include KOH and NaOH however other agents capable of increasing the pH of the media above about pH 7 may be used as well. The concentration of alkaline agents was about 0.05-2.0M, preferably about 0.1-1.0M. It is evident to the skilled person that these ranges include all the values falling within the explicitly recited ranges, such as e.g. 0.15-0.75M, 0.2-0.5M or 0.3-0.4M. The mixture was incubated at 30-100°C, for a period of 0.5-48hrs, preferably about 1-24 hrs. Again, it is appreciated by the skilled person that these ranges include all the values falling within the explicitly recited ranges, such as e.g. 40-80°C or 55-75°C as well as e.g. 1-40hrs, 2-35hrs, 5-30hrs, 10-25hrs but also 12-48hrs, 20-48hrs, 30-48hrs and 1-24hrs, 1-12hrs, 1-6hrs etc.. As a result reb *A* is hydrolyzed to reb *B.* The molar yield of conversion of reb *B* is about 5-100%, preferably about 10-90%.

After the reaction, the alkaline agent is neutralized by an acid, preferably by sulfuric acid or ortho-phosphoric acid until a pH of 3.0-4.0 is reached. Prior to adjusting the pH, or simultaneously, the mixture is cooled to about 10-30°C, this range including e.g. 15-25°C and 18-22°C. Upon neutralization, a precipitate is formed. The precipitate is separated by any method known in the art such as filtration or centrifugation and washed with water until the water reaches a pH of about 4.0-5.0. The obtained crystalline material is dried under vacuum at about 60-105°C to yield a mixture of reb *A* and reb *B* with ratio of about 5%:95% to about 95%:5%, preferably about 50%:50% to about 90%:10%. Again, it is appreciated by the skilled person that these ranges include all the values falling within the explicitly recited ranges, such as e.g. 70-95°C or 75-85°C as well as e.g. ratios of 10-80% to 80-10% or 20-70% to 70-20% etc..

In another embodiment, the separated precipitate is suspended in water and the mixture is subjected to continuous agitation over about 0.5-24 hrs, preferably about 1-3 hours, at about 50-100°C, preferably about 60-80°C. The ratio of precipitate to water (w/v) is about 1:5 to about 1:20, preferably about 1:10 to about 1:15. The washed crystals are separated and dried under vacuum at about 60-105°C to yield reb B with about 99% purity. These ranges include also e.g. 2-12 hrs or 5-10hrs of suspension; drying under vacuum at 70-100°C or 80-90°C etc., as will be apparent to the skilled person.

In one embodiment the obtained compositions have a water solubility of less than about 0.2% (w/v). The compositions were combined with the water at ratio of about1:1 (w/w) and the obtained mixture was further subjected to a gradient heat treatment which resulted in a high stability and high concentration solution. The gradient of about 1°C per minute was used in heating the mixture. The mixture was heated to the temperature of about 110-140°C, preferably about 118-125°C and was held at maximum temperature for about 0-120 min, preferably about 50-70 min. After the heat treatment, the solution was cooled down to room temperature at gradient of about 1°C per minute. Again it will be appreciated by the skilled person that the gradient may also be of e.g. 1.1°C per minute or 0.9°C per minute etc. The solution was spray dried by a laboratory spray dryer operating at about 175°C inlet and about 100°C outlet temperatures. An amorphous form of the composition was obtained with greater than about 20% solubility in water at room temperature.

The compositions can be used as sweetness enhancer, flavor enhancer and sweetener in various food and beverage products. Non-limiting examples of food and beverage products include carbonated soft drinks, ready to drink beverages, energy drinks, isotonic drinks, low-calorie drinks, zero-calorie drinks, sports drinks, teas, fruit and vegetable juices, juice drinks, dairy drinks, yoghurt drinks, alcohol beverages, powdered beverages, bakery products, cookies, biscuits, baking mixes, cereals, confectioneries, candies, toffees, chewing gum, dairy products, flavored milk, yoghurts, flavored yoghurts, cultured milk, soy sauce and other soy base products, salad dressings, mayonnaise, vinegar, frozen-desserts, meat products, fish-meat products, bottled and canned foods, tabletop sweeteners, fruits and vegetables.

Additionally the compositions can be used in drug or pharmaceutical preparations and cosmetics, including but not limited to toothpaste, mouthwash, cough syrup, chewable tablets, lozenges, vitamin preparations, and the like.

The compositions can be used "as-is" or in combination with other sweeteners, flavors and food ingredients.

Non-limiting examples of sweeteners include steviol glycosides, stevioside, rebaudioside *A,* rebaudioside *B,* rebaudioside *C*, rebaudioside *D,* rebaudioside *E*, rebaudioside *F*, dulcoside *A*, steviolbioside, rubusoside, as well as other steviol glycosides found in *Stevia rebaudiana* Bertoni plant and mixtures thereof, stevia extract, Luo Han Guo extract, mogrosides, high-fructose corn syrup, corn syrup, invert sugar, fructooligosaccharides, inulin, inulooligosaccharides, coupling sugar, maltooligosaccharides, maltodextins, corn syrup solids, glucose, maltose, sucrose, lactose, aspartame, saccharin, sucralose, sugar alcohols.

Non-limiting examples of flavors include lemon, orange, fruit, banana, grape, pear, pineapple, bitter almond, cola, cinnamon, sugar, cotton candy, vanilla flavors.

Non-limiting examples of other food ingredients include flavors, acidulants, organic and amino acids, coloring agents, bulking agents, modified starches, gums, texturizers, preservatives, antioxidants, emulsifiers, stabilisers, thickeners, gelling agents.

The following examples illustrate various embodiments of the invention.

### EXAMPLE 1

### Preparation of stevia composition

100g of rebaudioside *A* produced by PureCircle Sdn. Bhd. (Malaysia), containing, 98.1% rebaudioside A, 0.3% stevioside, 0.2% rebaudioside C, 0.2% rebaudioside F, 0.4% rebaudioside B and 0.6% rebaudioside D was dispersed in 1000mL aqueous KOH (1M) and incubated at 50°C for 2 hours. The mixture temperature was decreased to 20°C and the pH was adjusted to pH 4.0 with sulfuric acid. The solution was held under moderate agitation conditions for 4 hours and a precipitate was formed. The precipitate was filtered and washed on the filter with 2000mL of water. The washed crystals were dried under vacuum to yield 86g material containing about 84% reb *A* and 16% reb *B.* The water solubility (at 25°C) of obtained material was about 0.2% (w/v).

### EXAMPLE 2

### Preparation of stevia composition

100g of rebaudioside *A* produced by PureCircle Sdn. Bhd. (Malaysia), containing, 98.1% rebaudioside A, 0.3% stevioside, 0.2% rebaudioside C, 0.2% rebaudioside F, 0.4% rebaudioside B and 0.6% rebaudioside D was dispersed in 1000mL aqueous KOH (1M) and incubated at 80°C for 5 hours. The mixture temperature was decreased to 20°C and the pH was adjusted to about pH 4.0 with sulfuric acid. The solution was held under moderate agitation conditions for 4 hours and a precipitate was formed. The precipitate was filtered and washed on the filter with 2000mL of water. The washed crystals were dried under vacuum to yield about 75g material containing about 9% reb *A* and about 91% reb *B.* The water solubility (at 25°C) of obtained material was about 0.1% (w/v).

### EXAMPLE 3

### Preparation of stevia composition

100g of rebaudioside A produced by PureCircle Sdn. Bhd. (Malaysia), containing, 98.1% rebaudioside A, 0.3% stevioside, 0.2% rebaudioside C, 0.2% rebaudioside F, 0.4% rebaudioside B and 0.6% rebaudioside D was dispersed in 1000mL aqueous KOH (1M) and incubated at 80°C for 7 hours. The mixture temperature was decreased to 20°C and the pH was adjusted to about pH 4.0 with sulfuric acid. The solution was held under moderate agitation conditions for 3-4 hours and a precipitate was formed. The precipitate was filtered and washed on the filter with 2000mL of water. The washed crystals were dried under vacuum to yield about 71g material containing about 99.1% reb *B.* The water solubility (at 25°C) of obtained material was about 0.1% (w/v).

### EXAMPLE 4

### Preparation of stevia composition

75g of material prepared according to EXAMPLE 2 was suspended in 1000mL water. The mixture temperature was increased to 70°C. The suspension was held under moderate agitation conditions for 4 hours. The crystals were filtered and dried under vacuum to yield about 65g material containing about 99.0% reb B. The water solubility (at 25°C) of obtained material was about 0.1% (w/v).

### EXAMPLE 5

### Preparation of soluble stevia composition

50 g material prepared according to EXAMPLE 1 was mixed with 50g of water and incubated in thermostatted oil bath. The temperature was increased at 1°C per minute to 121°C. The mixture was maintained at 121°C for 1 hour and then the temperature was decreased to room temperature (25°C) at 1°C per minute. The solution was dried using YC-015 laboratory spray dryer (Shanghai Pilotech Instrument & Equipment Co. Ltd., China) operating at 175°C inlet and 100°C outlet temperature. About 47g of an amorphous powder was obtained with about 25% (w/v) solubility in water (at 25°C).

### EXAMPLE 6

### Preparation of soluble stevia composition

42 g of reb *A* produced by PureCircle Sdn. Bhd. (Malaysia) with purity of 99.2% (dry basis) and 8 g of reb *B* prepared according to EXAMPLE 4 were mixed with 50g of water and incubated in thermostatted oil bath. The temperature was increased at 1°C per minute to 121°C. The mixture was maintained at 121°C for 1 hour and then the temperature was decreased to room temperature (25°C) at 1°C per minute. The solution was dried using YC-015 laboratory spray dryer (Shanghai Pilotech Instrument & Equipment Co. Ltd., China) operating at 175°C inlet and 100°C outlet temperature. About 48g of an amorphous powder was obtained with about 1.5% (w/v) solubility in water (at 25°C).

### EXAMPLE 7

### Low-calorie orange juice drink

Orange concentrate (35%), citric acid (0.35%), ascorbic acid (0.05%), orange red color (0.01%), orange flavor (0.20%), and 0.05% stevia composition, were blended and dissolved completely in water (up to 100%) and pasteurized. The stevia composition was selected from a commercial stevia extract (containing stevioside 26%, rebaudioside *A* 55%, and 16% of other glycosides), a commercial rebaudioside A (containing 98.2% reb *A*) or material obtained according to EXAMPLE 5.

The sensory evaluations of the samples are summarized in Table 1. The data shows that the best results can be obtained by using the composition obtained according to EXAMPLE 5. Particularly the drinks prepared with said composition exhibited a rounded and complete flavor profile and mouthfeel.

**Table 1**

| Evaluation of orange juice drink samples | | | |
|---|---|---|---|
| Sample | Comments | | |
| | Flavor | Aftertaste | Mouthfeel |
| Stevia Extract | Sweet, licorice notes | Bitterness and aftertaste | Not acceptable |
| Reb *A* | Sweet, slight licorice notes | Slight bitterness and aftertaste | Not acceptable |
| EXAMPLE 5 | High quality sweetness, pleasant taste similar to sucrose, rounded and balanced flavor | Clean, no bitterness and no aftertaste | Full |

The same method can be used to prepare juices and juice drinks from other fruits, such as apples, lemons, apricots, cherries, pineapples, mangoes, etc.

### EXAMPLE 8

### Zero-calorie carbonated beverage

A carbonated beverage according to formula presented below was prepared.

| Ingredients | Quantity, % | | |
|---|---|---|---|
| | Stevia Extract | Reb *A* | EXAMPLE 5 |
| Cola flavor | 0.340 | 0.340 | 0.340 |
| ortho-Phosphoric acid | 0.100 | 0.100 | 0.100 |
| Sodium citrate | 0.310 | 0.310 | 0.310 |
| Sodium benzoate | 0.018 | 0.018 | 0.018 |
| Citric acid | 0.018 | 0.018 | 0.018 |
| Stevia composition | 0.050 | 0.050 | 0.050 |
| Carbonated water | to 100 | to 100 | to 100 |

The sensory properties were evaluated by 20 panelists. The results are summarized in Table 2.

**Table 2**

| Evaluation of zero-calorie carbonated beverage samples | | | |
|---|---|---|---|
| Taste attribute | Number of panelists detected the attribute | | |
| | Stevia Extract | Reb *A* | EXAMPLE 5 |
| Bitter taste | 15 | 10 | 0 |
| Astringent taste | 16 | 9 | 0 |
| Aftertaste | 14 | 12 | 0 |
| Comments | | | |
| Quality of sweet taste | Bitter aftertaste (15 of 20) | Bitter aftertaste (10 of 20) | Clean (20 of 20) |
| Overall evaluation | Satisfactory (1 of 20) | Satisfactory (5 of 20) | Satisfactory (20 of 20) |

The above results show that the beverages prepared using the composition obtained according to EXAMPLE 5 possessed the best organoleptic characteristics.

### EXAMPLE 9

### Diet cookies

Flour (50.0%), margarine (30.0%) fructose (10.0%), maltitol (8.0%), whole milk (1.0%), salt (0.2%), baking powder (0.15%), vanillin (0.1%) and different stevia compositions (0.03%) were kneaded well in dough-mixing machine. The obtained dough was molded and baked in oven at 200°C for 15 minutes. The stevia compositions were selected from a commercial stevia extract (containing stevioside 26%, rebaudioside A 55%, and 16% of other glycosides), a commercial rebaudioside A (containing 98.2% reb A) and material obtained according to EXAMPLE 5.

The sensory properties were evaluated by 20 panelists. The best results were obtained in samples containing the composition obtained according to EXAMPLE 5. The panelists noted a rounded and complete flavor profile and mouthfeel.

### EXAMPLE 10

### Yoghurt

Different stevia compositions (0.03%) and sucrose (4%) were dissolved in low fat milk. The stevia compositions were selected from a commercial stevia extract (containing stevioside 26%, rebaudioside A 55%, and 16% of other glycosides), a commercial rebaudioside *A* (containing 98.2% reb *A*) and the material obtained according to EXAMPLE 5. After pasteurizing at 82°C for 20 minutes, the milk was cooled to 37°C. A starter culture (3%) was added and the mixture was incubated at 37°C for 6 hours then at 5°C for 12 hours.

The sensory properties were evaluated by 20 panelists. The best results were obtained in samples containing the composition obtained according to EXAMPLE 5. The panelists noted a rounded and complete flavor profile and mouthfeel.

## Claims

1. A process for producing a rebaudioside B composition, comprising the steps of:
providing a stevia sweetener comprising rebaudioside A;
providing an alkaline aqueous solution free of any co-solvents;
hydrolyzing the rebaudioside A to rebaudioside B by dispersing the stevia sweetener in the alkaline solution and incubating for 0.5 to 48 hours at 30-100°C to form a mixture;
cooling the mixture to 10-30°C and adjusting the pH with acid to pH 3.0-4.0;
incubating the mixture at low temperature to obtain a precipitate;
separating the precipitate and washing it with water;
drying the washed precipitate to obtain the rebaudioside B composition.

2. The process of claim 1 further comprising the steps of:
suspending the rebaudioside B composition in water and incubating at 50-100°C for 0.5-24 hours;
separating the rebaudioside B composition from the water and drying the rebaudioside B composition to obtain a purified rebaudioside B composition;
wherein the purified rebaudioside B composition comprises rebaudioside B with greater than 99% purity.

3. The process of claim 1 or 2 further comprising the steps of:
suspending the rebaudioside B composition in water to form a suspension;
increasing the temperature of the suspension by a gradient heating method;
holding the suspension at 110-140°C;
decreasing the temperature of the suspension by a gradient cooling method to obtain a high stability and high concentration rebaudioside B composition solution; and
spray drying the high stability and high concentration rebaudioside B composition solution to provide an amorphous, highly soluble rebaudioside B composition powder.

4. The process of claim 3, wherein the rebaudioside B composition comprises a mixture of rebaudioside A and rebaudioside B at a ratio of 5%:95% to 95%:5% (w/w).

## Patentansprüche

1. Verfahren zum Herstellen einer Rebaudiosid B Zusammensetzung, umfassend die Schritte:
Bereitstellen eines Stevia-Süßstoffes umfassend Rebaudiosid A;
Bereitstellen einer alkalischen, wässrigen Lösung, die frei von jeglichen weiteren Lösungsmitteln ist;
Hydrolysieren des Rebaudiosids A zu Rebaudiosid B durch Dispergieren des Stevia-Süßstoffes in der alkalischen Lösung und Inkubieren für 0,5 bis 48 Stunden bei 30 bis 100 °C, um ein Gemisch zu bilden;
Kühlen des Gemisches auf 10 bis 30 °C und Anpassen des pH-Wertes mit Säure auf einen pH-Wert von 3,0 bis 4,0;
Inkubieren des Gemisches bei niedriger Temperatur, um ein Präzipitat zu erhalten;
Abtrennen des Präzipitats und Waschen desselben mit Wasser;
Trocknen des gewaschenen Präzipitats, um eine Rebaudiosid B Zusammensetzung zu erhalten.

2. Verfahren nach Anspruch 1, des Weiteren umfassend die Schritte:
Suspendieren der Rebaudiosid B Zusammensetzung in Wasser und Inkubieren bei 50 bis 100 °C für 0,5 bis 24 Stunden;
Abtrennen der Rebaudiosid B Zusammensetzung vom Wasser und Trocknen der Rebaudiosid B Zusammensetzung, um eine aufgereinigte Rebaudiosid B Zusammensetzung zu erhalten;
wobei die aufgereinigte Rebaudiosid B Zusammensetzung Rebaudiosid B mit mehr als 99% Reinheit umfasst.

3. Verfahren nach Anspruch 1 oder 2, des Weiteren umfassend die Schritte:
Suspendieren der Rebaudiosid B Zusammensetzung in Wasser, um eine Suspension zu bilden;
Erhöhen der Temperatur der Suspension durch ein Gradienten-Heiz-Verfahren;
Halten der Suspension bei 110 bis 140 °C;
Senken der Temperatur der Suspension durch ein Gradienten-Kühl-Verfahren, um eine hochstabile und hoch-konzentrierte Rebaudiosid B Zusammensetzungslösung zu erhalten; und
Sprühtrocknen der hoch-stabilen und hoch-konzentrierten Rebaudiosid B Zusammensetzungslösung, um ein amorphes, stark lösliches Rebaudiosid B Zusammensetzungspulver zu erhalten.

4. Verfahren nach Anspruch 3, wobei die Rebaudiosid B Zusammensetzung ein Gemisch aus Rebaudiosid A und Rebaudiosid B in einem Verhältnis von 5:95 Gew.-% bis 95:5 Gew.-% umfasst.

## Revendications

1. Procédé pour produire une composition de rébaudioside B, comprenant les étapes suivantes :
obtention d'un édulcorant de type stévia comprenant du rébaudioside A ;
obtention d'une solution aqueuse alcaline exempte de quelconques co-solvants ;
hydrolyse du rébaudioside A en rébaudioside B par dispersion de l'édulcorant de type stévia dans la solution alcaline et incubation pendant 0,5 à 48 heures à 30-100 °C pour qu'il se forme un mélange ;
refroidissement du mélange à 10-30°C et ajustement du pH avec un acide jusqu'à pH 3,0-4,0 ;
incubation du mélange à basse température pour que soit obtenu un précipité ;
séparation du précipité et lavage à l'eau de celui-ci ;
séchage du précipité lavé pour que soit obtenue la composition de rébaudioside B.

2. Procédé selon la revendication 1, comprenant en outre les étapes suivantes :
mise en suspension de la composition de rébaudioside B dans de l'eau et incubation à 50-100 °C pendant 0,5 à 24 heures ;
séparation de la composition de rébaudioside B d'avec l'eau et séchage de la composition de rébaudioside B pour que soit obtenue une composition de rébaudioside B purifiée ;
dans lequel la composition de rébaudioside B purifiée comprend de la rébaudioside B avec une pureté supérieure à 99 %.

3. Procédé selon la revendication 1 ou 2, comprenant en outre les étapes suivantes :
mise en suspension de la composition de rébaudioside B dans de l'eau pour qu'il se forme une suspension ;
augmentation de la température de la suspension par un procédé de chauffage à gradient ;
maintien de la suspension à 110-140 °C ;
abaissement de la température de la suspension par un procédé de refroidissement à gradient pour que soit obtenue une solution de composition de rébaudioside B fortement concentrée et très stable ; et
séchage par pulvérisation de la solution de composition de rébaudioside B fortement concentrée et très stable pour donner une poudre de composition de rébaudioside B amorphe et très soluble.

4. Procédé selon la revendication 3, dans lequel la composition de rébaudioside B comprend un mélange de rébaudioside A et de rébaudioside B en un rapport de 5 % / 95 % à 95 % / 5 % (p/p).
